# EUROPEAN PATENT SPECIFICATION

(11) **EP 2 097 123 B1**
(45) Date of publication and mention of the grant of the patent: **05.12.2012**
(21) Application number: 07863189.2
(22) Date of filing: 20.12.2007
(51) Int. Cl.: A61B 5/153

(54) **Vented phlebotomy needle with flashback chamber**
Belüftete Phlebotomienadel mit Rückflusskammer
Aiguille de phlébotomie ventilée ayant une chambre de retour

(30) Priority: 29.12.2006 US 877937 P
(43) Date of publication of application: 09.09.2009
(73) Proprietor: Tyco Healthcare Group LP, Mansfield, MA 02048 (US)
(72) Inventor: WEILBACHER, Eugene, E., Chesterfield, MT 63005 (US); SACCHETTI, Anthony, J., Mansfield, MA 02048 (US); NOBLE, Michael, J., St. Charles, MO 63304 (US)
(74) Representative: Gray, James
(86) International application number: PCT/US2007/026111
(87) International publication number: WO 2008/085393

(56) References cited:
- EP-A1- 0 619 096
- JP-A- 2000 166 903
- US-A- 4 365 630
- US-A- 4 416 290
- US-A- 5 303 713
- US-A1- 2005 245 869
- US-A1- 2005 283 093
- US-B2- 6 905 483

## Description

### BACKGROUND

### 1. Technical Field

The present disclosure relates to medical needles having structure to facilitate visualization of flashback. More specifically, the present disclosure relates to vented phlebotomy needles including structure to facilitate visualization of flashback.

### 2. Background of Related Art

Medical devices are well known for drawing blood from patients. These devices include standard needle-syringes, butterfly needle sets and phlebotomy needles. Typically, a butterfly needle set includes a hollow needle having a sharpened distal end and a proximal end which is secured to a needle hub. A proximal portion of the needle hub is connected to flexible tubing. The needle hub defines a fluid conduit communicating with the tubing and includes a pair of flexible, radially extending wings which facilitate grasping of the butterfly needle set by medical personnel. Generally, the flexible tubing is formed of a transparent material which allows medical personnel to visualize blood flow, i.e, flashback, through the tubing immediately proximal to the needle hub. Visualization of flashback allows medical personnel to confirm that the needle has been properly inserted into a patient.

Some clinicians utilize hypodermic needle-syringes with transparent needle hubs to obtain blood samples. During insertion, those clinicians typically observe flashback in the needle hubs.

Generally, phlebotomy needles have not included structure for visualizing flashback. Although the lack of structure for visualizing flashback in phlebotomy needles is not a major drawback for more experienced medical personnel, for those having little experience drawing blood with phlebotomy needles, the lack of any means to confirm that the needle has been properly positioned within a patient may increase the time required to draw blood and add to the discomfort of a patient.

In an attempt to overcome the above disadvantages, U.S. Patent No. 5,450,856 to Norris discloses a phlebotomy needle which is attachable to a blood collection tube and includes an outboard needle, an inboard needle and a bulb therebetween. The bulb is clear and allows medical personnel to visualize blood within the bulb when the outboard needle has been properly positioned within the vein of a patient. The bulb also includes a button which can be depressed by medical personnel to vent air from within the needle. Air within the needle prevents blood from flowing through the needle and must be vented.

Although the Norris phlebotomy needle facilitates visualization of flashback, a less expensive, less complex phlebotomy needle which facilitates visualization of flashback is desired.

US4416290 discloses a multiple liquid sample needle assembly which utilizes a housing having a porous filter which is gas permeable and liquid impermeable for providing gas displacement venting of gas displaced by a liquid sample received in the housing.

### SUMMARY

The present disclosure is directed to a phlebotomy needle comprising:
a needle including a distal needle portion having a sharpened distal end configured to pierce tissue and a proximal needle portion having a sharpened proximal end configured to pierce a stopper of a blood collection tube, the distal needle portion and the proximal needle portion defining a fluid channel;
a housing supported on the needle, the housing including a passive vent, wherein the housing defines a viewing region for visualizing blood in the housing; and
an elastomeric shield positioned about the proximal needle portion, the elastomeric shield and the proximal needle portion defining a fluid flow path into the housing, characterized in that the needle further includes a fluted member positioned within the housing, the fluted member defining a plurality of fluid channels, and in that the needle further includes a hydrophilic material supported within the housing.

### Brief Description Of An Embodiment

An embodiment of the presently disclosed vented phlebotomy needle with flashback chamber are disclosed herein with reference to the drawings, wherein:
FIG. 1 is a perspective view with parts separated of a "Prior Art" phlebotomy needle with a needle holder, protective needle covers and a blood collection tube shown in phantom;
FIG. 1A is a side cross-sectional view of the "Prior Art" phlebotomy needle, the needle holder, and the blood collection tube shown in phantom, assembled, with a distal portion of the phlebotomy needle positioned within a vein;
FIG. 2 is a side perspective view of a phlebotomy needle;
FIG. 2A is a cross-sectional view of the phlebotomy needle shown in FIG. 2;
FIG. 2B is a cross-sectional view taken along section lines 2B-2B of FIG. 2A;
FIG. 2C is a side cross-sectional view of another phlebotomy needle;
FIG. 3 is a side cross-sectional view of an alternate phlebotomy needle;
FIG. 3A is a cross-sectional view taken along section lines 3A-3A of FIG. 3;
FIG. 4 is a side perspective cutaway view of the presently disclosed phlebotomy needle;
FIG. 4A is a cross-sectional view taken along section lines 4A-4A of FIG. 4;
FIG. 5 is a side perspective cutaway view of another phlebotomy needle;
FIG. 6 is a side cross-sectional view of an embodiment of the presently disclosed phlebotomy needle; and
FIG. 7 is a cross-sectional view taken along section lines 7-7 of FIG. 6.

### Detailed Description Of Embodiments

An embodiment of the presently disclosed vented phlebotomy needle with flashback chamber will now be described in detail with reference to the drawings wherein like reference numerals designate identical or corresponding elements in each of the several views.

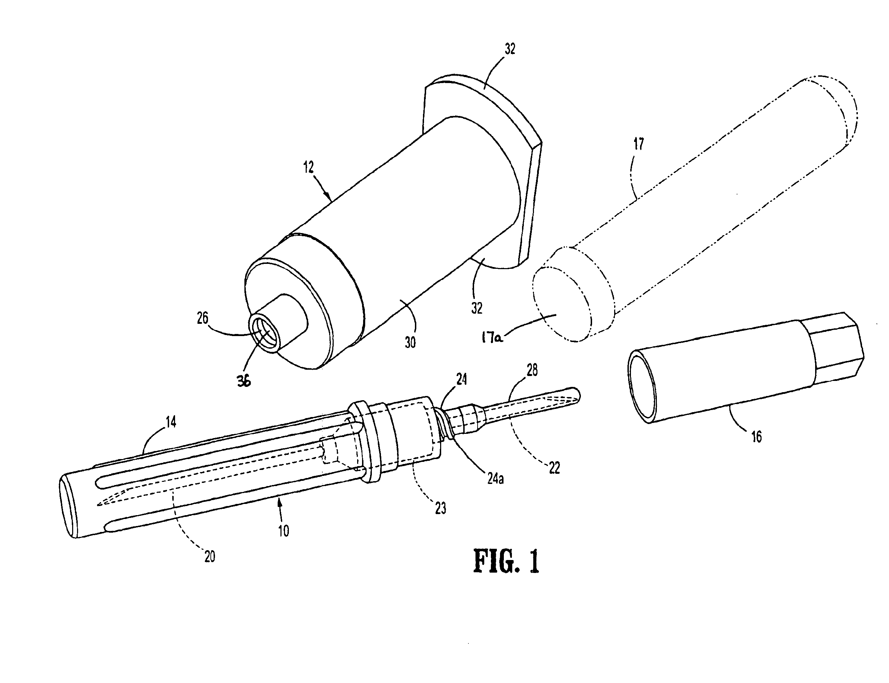

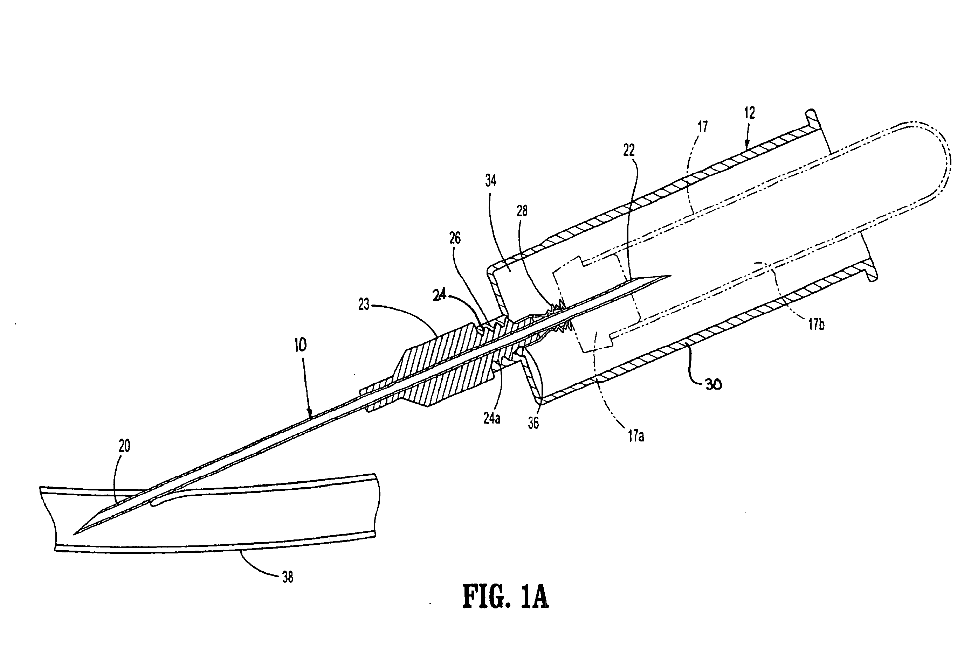

FIG. 1 illustrates a known phlebotomy needle 10, a needle holder 12, and needle covers 14 and 16. A blood collection tube 17 is shown in phantom. Phlebotomy needle 10 includes a distal needle portion 20, a proximal needle portion 22 and an intermediate needle hub 23 with engagement portion 24. Engagement portion 24 is provided to facilitate connection of phlebotomy needle 10 to needle holder 12. In one embodiment, the engagement portion includes a male fitting 24a which is configured to engage a female fitting or engagement member 26 provided on the distal end of needle holder 12. Alternately, other engagement structures including screw threads, snap-type connectors, etc., can be used. An elastomeric shield 28 is positioned over proximal needle portion 22 to seal the proximal needle portion 22 as will be described in further detail below.

Needle holder 12 includes a substantially cylindrical body 30 having a distal end including engagement member 26 and a proximal end including a pair of flange members 32. Flange members 32 facilitate gripping and insertion of a blood collection tube 17 by medical personnel. Cylindrical body 30 defines a cavity 34 (FIG. 1A) for receiving blood collection tube 17. Typically, blood collection tube 17 is sealed with a pierceable stopper 17a and defines a cavity 17b (FIG. 1A) of low pressure. The distal end of holder 12 defines an opening 36 through engagement member 26 which is dimensioned to receive proximal needle portion 22.

As shown in FIG. 1A, in use, needle 10 is secured to the distal end of needle holder 12 via engagement portion 24 and engagement member 26. When needle 10 is secured to needle holder 12, proximal needle portion 22 is positioned within cavity 34 of body 30 of needle holder 12 with elastomeric shield 28 positioned over proximal needle portion 22. Next, a blood collection tube 17 (shown in phantom) which includes pierceable stopper 17a is positioned within cavity 34. When collection tube 17 is inserted into cavity 34, proximal needle portion 22 engages stopper 17a and pierces through shield 28. Shield 28 is compressed downwardly (FIG. 1A) such that proximal needle portion 22 pierces stopper 40 and enters cavity 17b of collection tube 17. Typically, collection tube 17 is maintained at a vacuum such that when distal needle portion 20 is properly positioned in a vein 38 of a patient and collection tube 17 is positioned within cavity 34, blood will flow through needle 10 into blood collection tube 17. When blood collection tube 17 is removed from needle holder 12, elastomeric shield 28, which is resilient, returns to its original configuration and covers the proximal needle portion 22 to seal proximal needle portion 22.

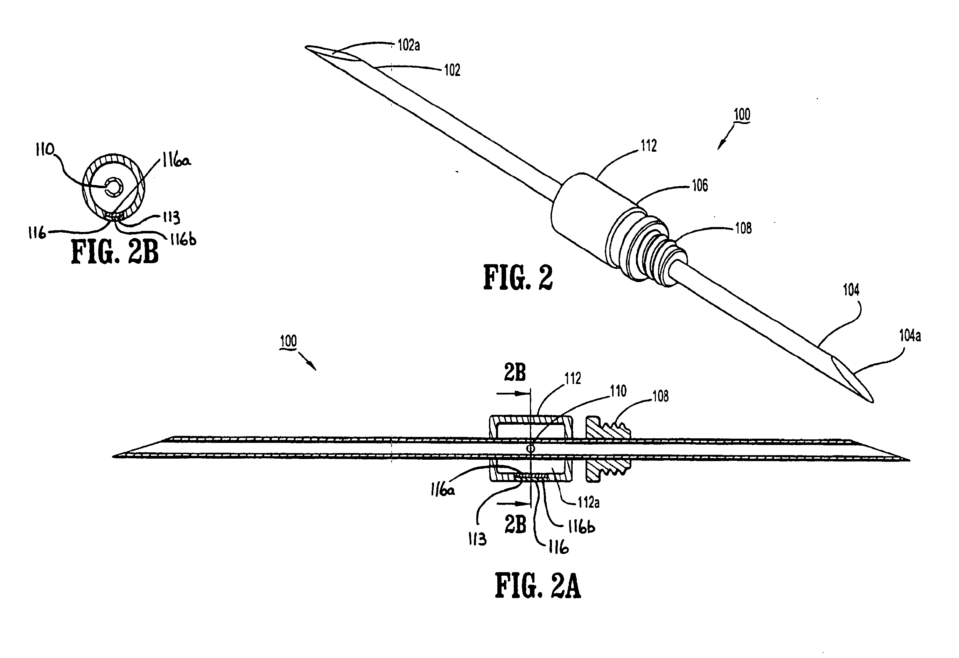

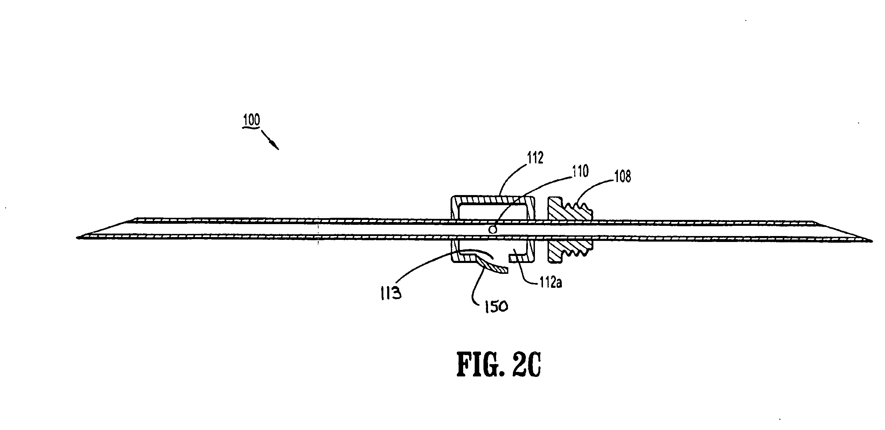

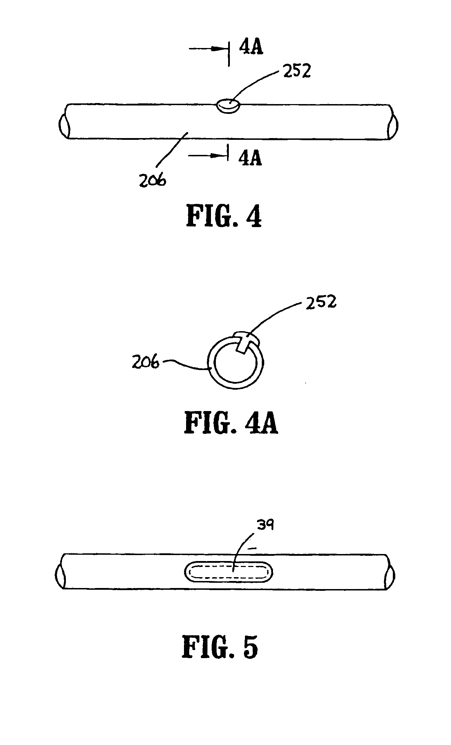

FIGS. 2 and 2A illustrate a phlebotomy needle with flashback chamber shown generally as 100. Needle 100 includes a distal end 102, a proximal end 104 and a central portion 106. Distal end 102 has a sharpened end 102a configured to pierce tissue of a patient and proximal end 104 has a sharpened end 104a configured to pierce the stopper 17a of a blood collection tube 17 (see FIG. 1 A). Central portion 106 includes an engagement member 108 as discussed above, configured to engage a needle holder (FIG. 1). Central portion 106 also includes an opening or hole 110 and a translucent or transparent housing 112 positioned about the portion of central portion 106 defining opening 110. Although opening 110 is illustrated as circular other configurations are envisioned e.g., rectangular, square, oval, an elongated slot 39 (FIG. 5), etc. Transparent housing 112 defines a blood reservoir 112a. A vent opening 113 preferably is formed in transparent housing 112. The vent opening 113 facilitates venting-of needle 100 to permit blood flow through needle 100. Vent opening 113 in housing 112 can be covered by a material which allows passage of air but does not permit passage of liquid, e.g., blood, as discussed below.

Referring to FIGS. 2A and 2B, vent opening 113 can be covered with a filter 116. Filter 116 preferably includes an inner layer 116a of hydrophilic material and an outer layer of hydrophobic material 116b. Preferably, at least one of layers 116a, 116b has a pore size of less than 0.45 microns. Such a pore size prevents entry of bacteria into housing 112 while allowing for passage of air.

Referring to FIG. 2C, alternatively, filter 116 can be replaced with a hydrophobic check valve, e.g., flap valve 150. Flap valve 150, as illustrated, is constructed to bend outwardly from housing 112 to uncover vent opening 113 to vent housing 112 when blood flow into housing 112 is initiated. A hydrophobic sheet 152 is positioned over opening 113 to prevent blood from exiting vent opening 113 when the check valve is open. Prior to the initiation of blood flow into housing 112, flap valve 150 seals opening 113 to prevent entry of material into housing 112. Alternately, flap valve 150 can be formed from a hydrophilic material if the pore size of the hydrophilic material is of a size to occlude passage of blood components.

Transparent housing 112 allows a medical practitioner to visualize the flow of blood through needle 100 when distal end 102 of needle 100 has been properly positioned within the vein of a patient, i.e., as blood flows through needle 100, air will be vented and blood will flow through opening 110 into reservoir 112a. This allows the medical practitioner to confirm that the distal end 102 of needle 100 has been properly positioned within a patient's vein to draw blood. This provides a great benefit to medical practitioners attempting to draw blood from a patient, especially those practitioners having a limited amount of experience drawing blood from a patient with a phlebotomy needle.

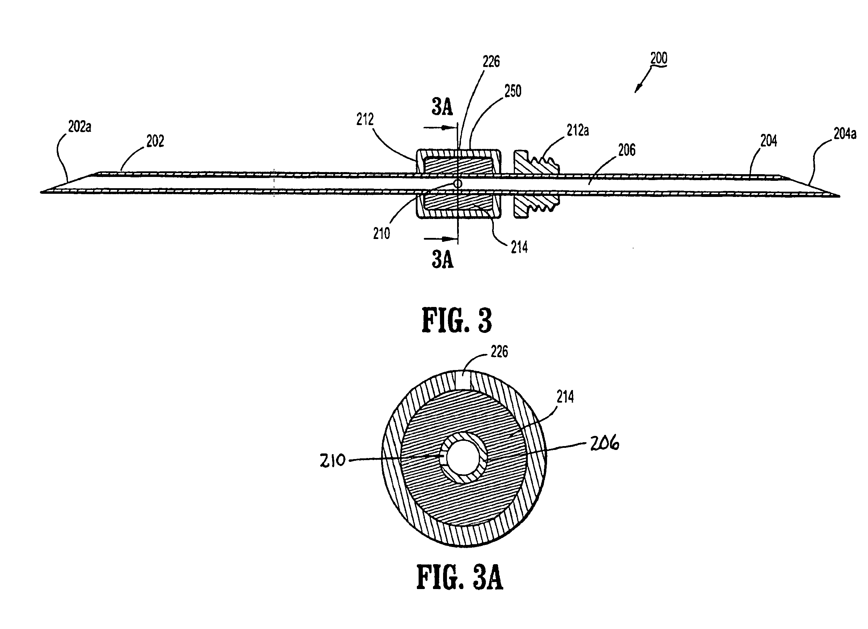

FIG. 3 illustrates a vented phlebotomy needle with flashback chamber shown generally as 200. Needle 200 is substantially similar to needle 100 except as will be described below. Needle 200 includes a distal end 202, a proximal end 204 and a central portion 206. Distal end 202 has a sharpened end 202a configured to pierce tissue of a patient and proximal end 204 has a sharpened end 204a configured to pierce the stopper of a blood collection tube (see FIG. 1A). Central portion 206 includes an opening or hole 210 and an engagement member 212a. A material 214 is positioned over hole 210. The material 214 can be a hydrophilic material that wicks blood into the material such that medical personnel can readily identify blood flow through needle 200. The material 214 can also be a hydrophilic material which is permeable to air but not to liquid, e.g., blood, to facilitate venting of needle 200. The hydrophilic material can be of the type which turns color when it is contacted by blood. The material can be in the form of a collar 250 (FIG. 3) or a plug 252 (FIG. 4). Further, the material can be positioned within a transparent housing or hub 212 or, alternately, positioned adjacent opening 210 without any housing. Alternatively, the material is a hydrophobic material which passes air but not blood. It is noted that a vent opening 226 can be provided in housing 212 to facilitate venting of the housing. The vent can be in the form of an opening in housing 212 which allows for air to flow from housing 212 as blood enters.

Alternatively, and as shown in FIGS. 3 and 3A, material 214 can be formed of a swellable material which prior to contacting blood allows for the passage of air through vent opening 226. Upon contacting blood, material 214 can swell to close the vent opening 226.

Although only hydrophilic and hydrophobic materials have been discussed herein, other materials which facilitate passage of air while preventing passage of blood and which provide an indication of blood flow can be used with the presently disclosed phlebotomy needle. The material can have other configurations not disclosed herein, e.g., the material may define a pad which covers the opening.

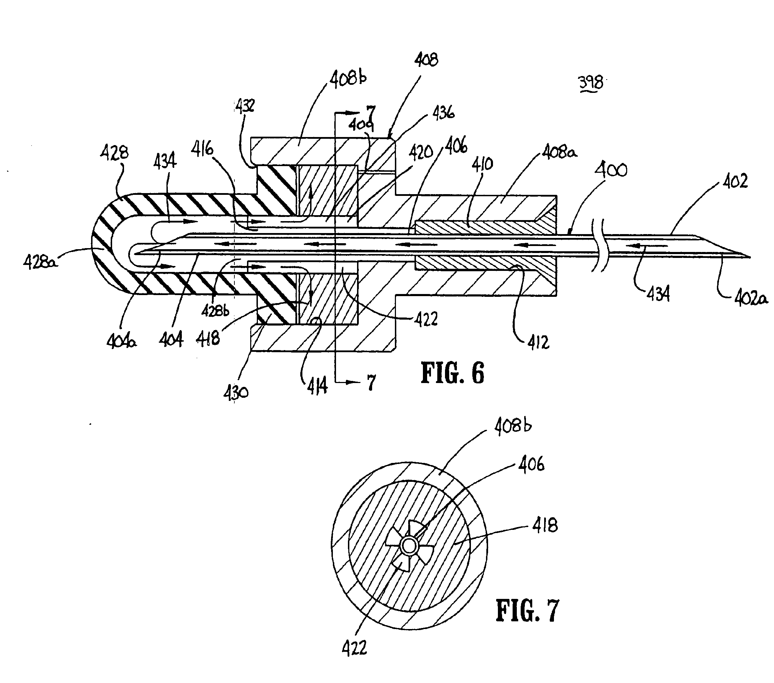

FIGS. 6 and 7 illustrate an embodiment of the presently disclosed phlebotomy needle with flashback chamber shown generally as device 398. Phlebotomy needle 398 includes a needle 400 and a stepped housing 408. Needle 400 includes a distal end 402, a proximal end 404 and a central portion 406. Distal end 402 has a sharpened end 402a configured to pierce tissue of a patient and proximal end 404 has a sharpened end 404a configured to pierce the stopper (not shown) of a blood collection tube (not shown).

A stepped housing 408 has a distal portion 408a and a larger-diameter proximal portion 408b. Stepped housing 408 is supported on central portion 406. Distal portion 408a defines a throughbore 412 dimensioned to receive needle central portion 406. Distal portion 408a is sealingly secured to needle 400 using, for example, an adhesive 410. Alternately, other known techniques can be used to secure needle 400 to housing 408, e.g., crimping, clamps, screws, welding etc. Proximal portion 408b is formed of a transparent or translucent material to facilitate visualization within proximal portion 408b of housing 408. Proximal portion 408b also defines throughbore 414 which communicates with the throughbore 412 but is larger in diameter than throughbore 412. Proximal portion 408b defines an annular recess 416 between portion 408b and proximal end 404 of needle 400. Recess 416 is dimensioned to receive a hydrophilic material 418 and an inner fluted member 420. Inner fluted member 420 is positioned about proximal end 404 of needle 400 and defines a plurality of channels 422 which communicate with hydrophilic material 418. A vent opening 409 is provided through housing 408.

An elastomeric shield 428 is positioned over proximal end 404 of needle 400. As discussed above, elastomeric shield 428 is resilient and functions to seal proximal end 404 of needle 400 when proximal end 404 of needle 400 is not connected to a blood collection tube (not shown). Elastomeric shield 428 includes a closed proximal end 428a and an open distal end 428b. Open distal end 428b includes an annular flange 430 which is positioned adjacent material 418 within recess 416 of housing 408. Proximal portion 408b of housing 408 includes an inwardly extending annular rib 432 which is positioned to engage flange 430 of elastomeric shield 428 to retain distal end 428b of shield 428 within recess 416 of housing 408. As illustrated by arrows 434, a flow path 436 is defined between elastomeric shield 428 and proximal end 404 of needle 400. Flow path 436 directs blood from proximal end 404 of needle 400 into fluted channels 422 of fluted member 420 into contact with hydrophilic material 418. In use, when blood contacts material 418, material 418 provides a visual indication of blood flow which is visible through proximal portion 408b of housing 408.

The vent openings 113 defined in connection with filter 116, FIGS. 2A-2B, with filter 252, FIGS. 4-4A, or with filter 39, FIG. 5, or with check valve 150, FIGS. 2C as well as opening 226, FIGS. 3-3A and opening 409, FIG. 6, all may be described as passive vent openings which automatically enable gases at greater than atmospheric pressure within the needle cannula to escape into the atmosphere. These passive vent openings do not require any action by a user of devices according to the present invention.

It will be understood that various modifications may be made to the embodiment disclosed herein. Therefore, the above description should not be construed as limiting, but merely as exemplifications of preferred embodiments. Those skilled in the art will envision other modifications within the scope of the claims appended hereto.

## Claims

1. A phlebotomy needle (398) comprising:
a needle (400 including a distal needle portion having a sharpened distal end (402a) configured to pierce tissue and a proximal needle portion having a sharpened proximal end (404a) configured to pierce a stopper of a blood collection tube, the distal needle portion and the proximal needle portion defining a fluid channel;
a housing (408) supported on the needle (400), the housing including a passive vent (409), wherein the housing (408) defines a viewing region for visualizing blood in the housing (408); and
an elastomeric shield (428) positioned about the proximal needle portion, the elastomeric shield (428) and the proximal needle portion defining a fluid flow path (436) into the housing (408), **characterized in that** the phlebotomy needle (398) further includes a fluted member (420) positioned within the housing (408), the fluted member (420) defining a plurality of fluid channels (422), and **in that** the phlebotomy needle (398) further includes a hydrophilic material (418) supported within the housing (408).

2. The phlebotomy needle (398) according to Claim 1, wherein the needle (400) has a central portion defining an opening into the housing (408).

## Patentansprüche

1. Eine Phlebotomienadel aufweisend:
- Eine Nadel (400) umfassend einen distalen Nadelbereich mit einem zum Durchstechen von Gewebe ausgebildeten angespitzten distalen Ende (402a) und einen proximalen Nadelabschnitt mit einem zum Durchstechen eines Stopfens eines Blutsammelröhrchens angespitzten proximalen Endes (404a), wobei der distale Nadelabschnitt und der proximale Nadelabschnitt einen Fluidkanal definieren;
- ein an der Nadel (400) abgestütztes Gehäuse (408), wobei das Gehäuse eine passive Entlüftung (409) umfasst, wobei das Gehäuse (408) einen Sichtbereich zum Sichtbarmachen von Blut im Gehäuse (408) definiert;
- einen um den proximalen Nadelbereich herum angeordneten elastomeren Schutz (428), wobei der elastomerer Schutz (428) und der proximale Nadelbereich einen Fluidströmungskanal (436) ins Gehäuse (408) bilden, **dadurch gekennzeichnet, dass** die Phlebotomienadel (398) des Weiteren ein kanneliertes Element (420) aufweist, welches im Gehäuse (408) angeordnet ist, wobei das kannelierte Element (420) eine Vielzahl von Fluidkanälen (422) definiert, und die Phlebotomienadel (398) des Weiteren ein im Gehäuse (408) abgestütztes hydrophiles Material (418) aufweist.

2. Phlebotomienadel (398) gemäß Anspruch 1, wobei die Nadel (400) eine zentrale Position aufweist, welche eine Öffnung ins Gehäuse (408) definiert.

## Revendications

1. Aiguille de phlébotomie (398) comprenant :
une aiguille (400) comprenant une partie d'aiguille distale présentant une extrémité distale acérée (402a) conçue pour percer le tissu et une partie d'aiguille proximale présentant une extrémité proximale acérée (404a) conçue pour percer le bouchon d'un tube de prélèvement sanguin, la partie d'aiguille distale et la partie d'aiguille proximale définissant un canal de fluide ;
un logement (408) supporté sur l'aiguille (400), le logement comprenant un évent passif (409), le logement (408) définissant une zone de visualisation pour voir le sang dans le logement (408) ; et
une chemise élastomère (428) située autour de la partie d'aiguille proximale, la chemise élastomère (428) et la partie d'aiguille proximale définissant un passage d'écoulement de fluide (436) dans le logement (408), **caractérisée en ce que** l'aiguille de phlébotomie (398) comprend, en outre, un élément cannelé (420) situé dans le logement (408), l'élément cannelé (420) définissant une pluralité de canaux de fluide (422), et **en ce que** l'aiguille de phlébotomie (398) comprend, en outre, un matériau hydrophile (418) supporté dans le logement (408).

2. Aiguille de phlébotomie (398) selon la revendication 1, dans laquelle l'aiguille (400) comporte une partie centrale définissant une ouverture dans le logement (408).
